# FASCICULE DE BREVET EUROPEEN

(11) **EP 2 976 162 B1**
(45) Date de publication et mention de la délivrance du brevet: **18.07.2018**
(21) Numéro de dépôt: 14720993.6
(22) Date de dépôt: 18.03.2014
(51) Int. Cl.: B05D 5/08, B05D 7/00

(54) **PROCÉDÉ DE TRAITEMENT DE SURFACE D'UNE VALVE DOSEUSE**
VERFAHREN ZUR OBERFLÄCHENBEHANDLUNG EINES DOSIERVENTILS
PROCESS FOR THE SURFACE TREATMENT OF A METERING VALVE

(30) Priorité: 19.03.2013 FR 1352433
(43) Date de publication de la demande: 27.01.2016
(73) Titulaire: Aptar France SAS, 27110 Le Neubourg (FR)
(72) Inventeur: BROUET, Guillaume, F-76000 Rouen (FR)
(74) Mandataire: CAPRI
(86) Numéro de dépôt international: PCT/FR2014/050620
(87) Numéro de publication internationale: WO 2014/147331

(56) Documents cités:
- EP-A1- 0 338 418
- WO-A1-2011/077056
- WO-A1-2013/106588

## Description

La présente invention concerne un procédé de traitement de surface pour des valves doseuses.

Les valves doseuses fonctionnent avec des gaz propulseurs, et sont utilisées notamment dans des dispositifs de distribution de produit fluide du type MDI (« Metered Dose Inhaler »).

Les dispositifs de distribution de produits fluides du type MDI sont bien connus. Ils comportent généralement un réservoir sur lequel est assemblée une valve doseuse comportant une chambre de valve, définissant la dose à distribuer à chaque actionnement, et une soupape se déplaçant dans ladite chambre de dosage lors de l'actionnement. La maîtrise des frottements de la soupape, qui peuvent occasionner des dysfonctionnements de la valve, est un enjeu majeur. En particulier dans le domaine pharmaceutique, les risques de dysfonctionnement de la valve peuvent être critiques, par exemple pour des traitements de crise, tels que l'asthme. Tout blocage ou grippage de la soupape du aux frottements est potentiellement préjudiciable.

Il est connu de limiter ce risque notamment en déposant du silicone, en particulier du dimethylpolysiloxane, sur les composants soumis à frottement. Cette dépose de silicone libre peut être effectuée par différentes techniques, tel que par exemple le tamponnage. Ce mode de lubrification par dépôt de silicone présente certains inconvénients. Ainsi, la tenue du silicone est limitée, notamment lors de l'opération de remplissage du produit fluide au travers de la valve, où un pourcentage important de ce silicone est alors entrainé (effet dit de lavage ou effet « karcher »). De plus, la tenue dans le temps du silicone sur les surfaces lubrifiées n'est pas garantie, ce qui peut conduire à des dysfonctionnements au bout d'un certain temps.

Il a aussi été proposé de greffer chimiquement du silicone sur des surfaces à traiter. Les documents WO 2010/112610, WO 2011/077055 et WO 2011/077056 décrivent notamment un tel procédé. L'efficacité de ce traitement de surface n'est toutefois pas optimale, et s'est avérée potentiellement insuffisante pour garantir la fiabilité des valves doseuses.

D'autres procédés de traitement de surface existants présentent également des inconvénients. Ainsi, certains procédés ne sont utilisables que sur des surfaces planes. D'autres procédés imposent un choix limité de substrat. La polymérisation de molécules induite par plasma est complexe, coûteuse, et la couche de revêtement obtenue est difficile à contrôler et présente des problèmes de vieillissement. De même, la polymérisation de molécules induite par ultraviolets est également complexe et coûteuse, et ne fonctionne qu'avec des molécules photosensibles. Il en est de même de la polymérisation radicalaire par transfert d'atomes (ATRP), qui est aussi complexe et coûteuse. Enfin, les procédés d'électro-greffage sont complexes et nécessitent des surfaces de support conductrices.

La présente invention a pour but de proposer un procédé de traitement de surface qui ne reproduit pas les inconvénients susmentionnés.

La présente invention a aussi pour but de proposer un procédé de traitement de surface qui améliore la fiabilité des valves doseuses, en limitant ou éliminant les risques de blocage de la soupape de valve.

En particulier, la présente invention a pour but de fournir un procédé de traitement de surface qui soit efficace, durable et simple à réaliser.

La présente invention a donc pour objet un procédé de traitement de surface d'une valve doseuse selon la revendication 1. Des modes de réalisation avantageux sont décrits dans les revendications dépendantes.

Selon l'invention, une étape de greffage de silicone est associée à une étape de dépôt de silicone libre, c'est-à-dire un silicone non greffé. La lubrification de la surface des composants traités est donc obtenue au moyen d'une couche d'un silicone à terminaison vinylique greffé sur ladite surface (silicone greffé), et d'un dépôt additionnel de silicone non greffé (silicone libre). Le silicone libre peut être différent du silicone greffé, mais de préférence ces deux silicones sont identiques à celui habituellement utilisé dans l'industrie pharmaceutique (dimethylpolysiloxane), afin notamment de conserver les mêmes caractéristiques de compatibilité chimique en contact avec le principe actif dans le réservoir. Un avantage de déposer un silicone libre identique à celui déjà habituellement utilisé est que son comportement au contact du fluide et principe actif, ce qui peut arriver par exemple en cas de migration partielle dans le réservoir, est connu. Ceci simplifie le respect des exigences réglementaires, notamment concernant la compatibilité avec le produit actif.

La tenue du silicone libre sur la couche de silicone greffé est améliorée par un phénomène chimique d'association des chaines siloxanes du silicone greffé et du silicone libre entre elles. Ce phénomène se produit afin de minimiser l'énergie de surface totale du silicone.

Ainsi, l'invention permet d'améliorer le siliconage des valves, et donc augmente leur fiabilité. En particulier, elle permet de réduire voire supprimer les risques de dysfonctionnement de la valve notamment en fin de vidange, c'est-à-dire lorsqu'il ne reste plus assez de silicone en surface pour assurer la fonction lubrification recherchée, ou lorsqu'un effort résistant à la remontée de la valve risque de perturber son fonctionnement, par exemple en cas du rajout d'un compteur à la valve dans le dispositif MDI.

### Silicone greffé:

Pour l'étape de greffage, on peut utiliser un procédé similaire à celui décrit dans le document WO 2008/078052, qui décrit un procédé de préparation d'un film organique à la surface d'un support solide dans des conditions non électrochimiques. Ce type de procédé s'est avéré adapté pour former un film mince anti-frottements sur des surfaces mobiles lors de l'actionnement des dispositifs de distribution susmentionnés.

De manière synthétique, le procédé a pour but de préparer un film mince de silicone à la surface du corps de valve et/ou de la soupape de valve. Ce procédé comprend principalement une mise en contact de ladite surface de support avec une solution liquide. Celle-ci comprend au moins un solvant et au moins un primaire d'adhésion permettant la formation d'entités radicalaires à partir du primaire d'adhésion.

Le film mince de silicone est lié de manière covalente à la surface du support sur lequel est effectué le procédé. Selon l'épaisseur du film, sa cohésion est assurée par les liaisons covalentes qui se développent entre les différentes unités.

Le solvant employé dans le cadre du procédé peut être de nature protique ou aprotique. Il est préférable que le primaire soit soluble dans ledit solvant.

Par "solvant protique" on entend un solvant qui comporte au moins un atome d'hydrogène susceptible d'être libéré sous forme de proton. Le solvant protique peut être choisi dans le groupe constitué par l'eau, l'eau désionisée, l'eau distillée, acidifiées ou non, l'acide acétique, les solvants hydroxylés comme le méthanol et l'éthanol, les glycols liquides de faible poids moléculaire tels que l'éthylèneglycol, et leurs mélanges. Dans une première variante, le solvant protique n'est constitué que par un solvant protique ou par un mélange de différents solvants protiques. Dans une autre variante, le solvant protique ou le mélange de solvants protiques peut être utilisé en mélange avec au moins un solvant aprotique, étant entendu que le mélange résultant présente les caractéristiques d'un solvant protique. L'eau acidifiée est le solvant protique préféré et, plus particulièrement, l'eau distillée acidifiée ou l'eau désionisée acidifiée.

Par "solvant aprotique" on entend un solvant qui n'est pas considéré comme protique. De tels solvants ne sont pas susceptibles de libérer un proton ou d'en accepter un dans des conditions non extrêmes. Le solvant aprotique est avantageusement choisi parmi la diméthylformamide (DMF), l'acétone et le diméthyl sulfoxyde (DMSO).

Le terme "primaire d'adhésion" correspond à toute molécule organique susceptible, sous certaines conditions, de se chimisorber à la surface de support par réaction radicalaire tel qu'un greffage chimique radicalaire. De telles molécules comportent au moins un groupe fonctionnel susceptible de réagir avec un radical et également une fonction réactive vis-à-vis d'un autre radical après chimisorption. Ces molécules sont ainsi capables de former un film de nature polymérique après greffage d'une première molécule à la surface du support puis réaction avec d'autres molécules présentes dans son environnement.

Le terme "greffage chimique radicalaire" se réfère notamment à l'utilisation d'entités moléculaires possédant un électron non apparié pour former des liaisons de type liaison covalente avec la surface de support, lesdites entités moléculaires étant générées indépendamment de la surface de support sur laquelle elles sont destinées à être greffées. Ainsi, la réaction radicalaire conduit à la formation de liaisons covalentes entre la surface de support concernée et le dérivé du primaire d'adhésion greffé puis entre un dérivé greffé et des molécules présentes dans son environnement.

Par "dérivé du primaire d'adhésion" on entend une unité chimique résultant du primaire d'adhésion, après que ce dernier a réagi par greffage chimique radicalaire notamment avec la surface de support, ou avec un autre radical. Il est clair pour l'homme du métier que la fonction réactive vis-à-vis d'un autre radical après chimisorption du dérivé du primaire d'adhésion est différente de la fonction impliquée dans la liaison covalente notamment avec la surface de support. Le primaire d'adhésion est de préférence un sel d'aryle clivable choisi dans le groupe constitué par les sels d'aryle diazonium, les sels d'aryle d'ammonium, les sels d'aryle phosphonium, les sels d'aryle sulfonium et les sels d'aryle iodonium.

Les sels d'aryles clivables sont choisis parmi les composés de formule générale ArN₂⁺, X⁻ dans laquelle Ar représente le groupe aryle et X⁻ représente un anion. Le groupe aryle dans un composé organique est un groupe fonctionnel issu d'un noyau aromatique.

Dans un mode de réalisation, les anions X⁻ sont choisis parmi les anions inorganiques tels que les halogénures, comme I⁻, Cl⁻ et Br⁻, les halogénoborates tels que le tetrafluoroborate et les anions organiques tels que les alcoolates, les carboxylates, les perchlorates et les sulfonates.

Dans un mode de réalisation, les groupes aryles Ar sont choisis parmi les aromatiques ou hétéroaromatiques, éventuellement mono- ou polysubstitués, constitués d'un ou plusieurs cycles aromatiques de 3 à 8 carbones. Les hétéroatomes des composés hétéroaomatiques sont choisis parmi N, O, P et S. Les substituants peuvent contenir des groupes alkyles et un ou plusieurs hétéroatomes tels que N, O, F, Cl, P, Si, Br ou S.

Dans un mode de réalisation, les groupes aryles sont choisis parmi les groupes aryles substitués par des groupements attracteurs tels que NO₂, COH, CN, CO₂H, les cétones, les esters, les amines et les halogènes.

Dans un mode de réalisation, les groupes aryles sont choisis dans le groupe constitué par le phényle et le nitrophényle.

Dans un mode de réalisation, le sel d'aryle clivable est choisi dans le groupe constitué par le tétrafluoroborate de phényldiazonium, le tétrafluoroborate de 4-nitrophényldiazonium, le tétrafluoroborate de 4-bromophényldiazonium, le chlorure de 4-aminophényldiazonium, le chlorure de 4-aminométhylphényldiazonium, le chlorure de 2-méthyl-4-chlorophényldiazonium, le tétrafluoroborate de 4-benzoylbenzènediazonium, le tétrafluoroborate de 4-cyanophényldiazonium, le tétrafluoroborate du 4-carboxyphényldiazonium, le tétrafluoroborate de 4-acétamidophényldiazonium, le tétrafluoroborate de l'acide 4-phénylacétique diazonium, le sulfate de 2-méthyl-4-[(2-méthylphényl)diazényl]benzènediazonium, le chlorure de 9,10-dioxo-9,10-dihydro-1-anthracènediazonium, le tétrafluoroborate de 4-nitronaphtalènediazonium et le tétrafluoroborate de naphtalènediazonium.

Dans un mode de réalisation, le sel d'aryle clivable est choisi dans le groupe constitué par le tétrafluoroborate de 4-nitrophényldiazonium, le chlorure de 4-aminophényldiazonium, le chlorure de 2-méthyl-4-chlorophényldiazonium, le tétrafluoroborate du 4-carboxyphényldiazonium.

Dans un mode de réalisation, la concentration en sel d'aryle clivable est comprise entre 5.10⁻³ M et 10⁻¹ M.

Dans un mode de réalisation, la concentration en sel d'aryle clivable est de l'ordre de 5.10⁻² M.

Dans un mode de réalisation, le sel d'aryle clivable est préparé in situ.

Avantageusement, ladite étape de greffage chimique est initiée par activation chimique d'un sel de diazonium pour former une couche d'ancrage pour ledit film mince.

Avantageusement, ladite étape de greffage chimique est initiée par activation chimique.

Dans un mode de réalisation ladite activation chimique est initiée par la présence d'un réducteur dans la solution.

Dans un mode de réalisation la solution comprend un agent réducteur.

Par agent réducteur, on entend un composé qui lors d'une réaction d'oxydoréduction cède des électrons. Selon un aspect de la présente invention, l'agent réducteur présente un potentiel d'oxydoréduction dont la différence de potentiel par rapport au potentiel d'oxydoréduction du sel d'aryle clivable est comprise entre 0,3 V et 3 V.

Selon un aspect de l'invention, l'agent réducteur est choisi dans le groupe constitué par les métaux réducteurs pouvant se présenter sous forme finement divisée tels que du fer, du zinc, ou du nickel, un sel métallique pouvant être sous forme de métallocène et un réducteur organique tel que l'acide hypophosphoreux, l'acide ascorbique.

Dans un mode de réalisation, la concentration en agent réducteur est comprise entre 0,005 M et 2 M.

Dans un mode de réalisation, la concentration en agent réducteur est de l'ordre de 0,6 M.

Dans un mode de réalisation, ledit film mince a une épaisseur inférieure à 1 micromètre, comprise entre 10 et 2000 angströms, avantageusement comprise entre 10 et 800 angströms, de préférence comprise entre 400 et 1000 angströms. Aucune technique de revêtement classique ne permet d'obtenir des couches aussi minces greffées chimiquement.

On entend par siloxane à terminaison vinylique ou acrylique un hydrure saturé de silicium et d'oxygène formé de chaines droites ou ramifiées, d'atomes de silicium et d'oxygène en alternance comportant des motifs vinyliques ou des motifs acryliques terminaux.

Dans un mode de réalisation les siloxanes à terminaison vinylique ou acrylique sont choisis dans le groupe constitué par les polyalkylsiloxanes à terminaisons acryliques ou vinyliques tels que le polyméthylsiloxane à terminaisons vinyliques ou acryliques, le polydiméthylsiloxane à terminaisons vinyliques ou acryliques comme le polydiméthylsiloxane-acrylate (PDMS-acrylate), les polyarylsiloxanes à terminaisons vinyliques ou acryliques tels que le polyphénylsiloxane à terminaisons vinyliques ou acryliques comme le polyvinylphénylsiloxane, les polyarylalkylsiloxanes à terminaisons vinyliques ou acryliques tels que le polyméthylphénylsiloxane à terminaisons vinyliques ou acryliques.

Dans un mode de réalisation une différence de potentiel est appliquée dans ladite solution.

On entend par différence de potentiel, la différence de potentiel d'oxydoréduction mesurée entre deux électrodes.

Dans un mode de réalisation, la différence de potentiel est appliquée par un générateur relié à deux électrodes, identiques ou différentes, plongeant dans la solution pendant l'étape de trempage.

Dans un mode de réalisation, les électrodes sont choisies parmi l'inox, l'acier, le nickel, le platine, l'or, l'argent, le zinc, le fer, le cuivre, sous forme pure ou sous forme d'alliage.

Dans un mode de réalisation, les électrodes sont en inox.

Dans un mode de réalisation, la différence de potentiel appliquée par un générateur est comprise entre 0,1 V et 2 V.

Dans un mode de réalisation, elle est de l'ordre de 0,7 V.

Dans un mode de réalisation, la différence de potentiel est générée par une pile chimique.

On entend par pile chimique une pile composée de deux électrodes reliées par un pont ionique. Selon la présente invention, les deux électrodes sont judicieusement choisies pour que la différence de potentiel soit comprise entre 0,1 V et 2,5 V.

Dans un mode de réalisation, la pile chimique est créée entre deux électrodes différentes plongeant dans la solution.

Dans un mode de réalisation, les électrodes sont choisies parmi le nickel, le zinc, le fer, le cuivre, l'argent sous forme pure ou sous forme d'alliage.

Dans un mode de réalisation, la différence de potentiel générée par la pile chimique est comprise entre 0,1 V et 1,5 V.

Dans un mode de réalisation, la différence de potentiel est de l'ordre de 0,7 V.

Dans un mode de réalisation, les électrodes sont isolées chimiquement pour éviter tout contact entre le substrat immergé dans la solution et les électrodes plongées également dans la solution.

L'exemple qui suit a été réalisés dans une cuve en verre. Sauf précision contraire, il a été réalisé dans des conditions normales de température et de pression (environ 22 °C sous environ 1 atm) à l'air ambiant. Sauf mention contraire, les réactifs employés ont été directement obtenus dans le commerce sans purification supplémentaire. Les échantillons ont au préalable subit un nettoyage sous ultrasons à l'eau savonneuse à 40 °C.

### Exemple - Greffage d'un film de poly(diméthylsiloxane) des pièces d'une valve pour la lubrifier

Par valve on entend un dispositif de distribution de produit fluide contenant du gaz propulseur, comportant un corps de valve dans lequel coulisse une soupape.

Le sodium dodécyle benzène sulfonate (1,307 g, 0,015 M) a été solubilisé dans 175 mL d'eau milliQ. Le poly(diméthylsiloxane) à terminaisons vinyliques (2,5 g, 10 g/L) a été ajouté puis le mélange a été mis à agiter magnétiquement pour former une émulsion.

L'acide 4-aminobenzoïque (3,462 g, 2,5 10⁻² mol) a été solubilisé dans une solution d'acide chlorhydrique (9,6 mL dans 20 ml d'eau MQ) et d'acide hypophosphoreux (33 mL, 3,1 10⁻¹ mol). Cette solution a été ajoutée à l'émulsion de PDMS.

A cette émulsion, ont été ajoutés 10 ml d'une solution de NaNO₂ (1,664 g, 2,37 10⁻² mol) dans l'eau MQ et les échantillons : joints EPDM ou Nitrile et haut de soupape en POM ainsi qu'une lame d'or témoin.

Après 15 minutes de réaction, les échantillons ont été retirés puis rincés successivement à l'eau Milli-Q ®, à l'éthanol ainsi qu'à l'hexane.

La présence de PDMS sur la lame d'or et les autres échantillons a été confirmée par analyses IR avec les bandes spécifiques du PDMS à 1260, 1110 et 1045 cm⁻¹.

### Silicone libre:

L'étape d'appliquer du silicone libre à la surface de support comportant du silicone greffé peut être réalisée conformément à l'art antérieur, en particulier via le tamponnage, le pan coating ou le procédé Xdot.

Le tamponnage prévoit, dans un premier temps, de déposer du silicone sur une surface S, et dans un second temps, de mettre cette surface S imbibée en contact avec la pièce à siliconer selon une cinématique appropriée. Il en résulte le transfert ou dépôt de silicone sur le composant à siliconer. Ce procédé fonctionne sur le principe d'un tampon ou d'un pinceau.

Le « pan coating » est un procédé qui prévoit de faire une répartition du silicone par brassage des pièces. Typiquement, on introduit dans un volume fermé plusieurs pièces et du silicone et on génère une cinématique pendant une durée donnée afin de répartir le silicone sur les pièces. Ce procédé fonctionne sur le principe d'une bétonnière.

Le procédé Xdot comporte des gouttelettes de silicones générées par une buse dont la fermeture peut être assurée par exemple par un élément piézo-électrique ; le silicone arrivant d'un réservoir sous pression, les micromouvement réalisés par l'obturation piézo-électrique permettent de générer des micro gouttelettes qui sont projetées par leur inertie sur la pièce à siliconer. Ce procédé fonctionne sur le principe d'un pistolet à peinture.

Diverses modifications sont également possibles pour un homme du métier sans sortir du cadre de la présente invention tel que défini par les revendications annexées.

## Revendications

1. Procédé de traitement de surface d'une valve doseuse, ladite valve doseuse comportant un corps de valve et une soupape coulissant dans ledit corps de valve, **caractérisé en ce que** ledit procédé comprend une première étape de former par greffage chimique un film mince de silicone sur au moins une surface de support dudit corps de valve et/ou de ladite soupape, et une seconde étape d'appliquer sur ledit film mince de silicone greffé du silicone libre, de sorte que ladite surface de support traitée comporte simultanément du silicone greffé et du silicone libre, les chaînes siloxanes du silicone greffé et du silicone libre s'associant chimiquement entre elles pour renforcer la tenue du silicone libre sur ledit film mince de silicone greffé, ledit film mince de silicone greffé ayant une épaisseur inférieure à 1 micromètre, de préférence comprise entre 10 et 2000 angströms.

2. Procédé selon la revendication 1, dans lequel ladite étape de greffage comprend la mise en contact de ladite surface en contact avec le produit fluide avec une solution comprenant au moins un primaire d'adhésion, ledit primaire d'adhésion étant un sel d'aryle clivable et au moins un monomère ou un polymère choisi dans le groupe constitué par les siloxanes à terminaison vinylique ou acrylique.

3. Procédé selon la revendication 2, dans lequel les siloxanes à terminaison vinylique ou acrylique sont choisis dans le groupe constitué par les polyalkylsiloxanes à terminaisons acryliques ou vinyliques tels que le polyméthylsiloxane à terminaisons vinyliques ou acryliques, le polydiméthylsiloxane à terminaisons vinyliques ou acryliques comme le polydiméthylsiloxane-acrylate (PDMS-acrylate), les polyarylsiloxanes à terminaisons vinyliques ou acryliques tels que le polyphénylsiloxane à terminaisons vinyliques ou acryliques comme le polyvinylphénylsiloxane, les polyarylalkylsiloxanes à terminaisons vinyliques ou acryliques tels que le polyméthylphénylsiloxane à terminaisons vinyliques ou acryliques.

4. Procédé selon la revendication 2 ou 3, dans lequel le sel d'aryle clivable est choisi dans le groupe constitué par les sels d'aryle diazonium, les sels d'aryle ammonium, les sels d'aryle phosphonium, les sels d'aryle sulfonium et les sels d'aryle iodonium.

5. Procédé selon l'une quelconque des revendications précédentes, dans lequel ladite seconde étape d'appliquer du silicone libre est réalisé par tamponnage.

## Patentansprüche

1. Verfahren zur Oberflächenbehandlung eines Dosierventils, wobei das Dosierventil ein Ventilgehäuse und ein in dem Ventilgehäuse gleitendes federgespanntes Ventil umfasst, **dadurch gekennzeichnet, dass** das Verfahren einen ersten Schritt des Bildens eines dünnen Silikonfilms auf mindestens einer Trägerfläche des Ventilgehäuses und/oder des federgespannten Ventils durch chemisches Aufpolymerisieren und einen zweiten Schritt des Aufbringens von freiem Silikon auf dem dünnen aufpolymerisierten Silikonfilm, so dass die behandelte Trägerfläche gleichzeitig aufpolymerisiertes und freies Silikon aufweist, umfasst, wobei sich die Siloxanketten des aufpolymerisierten Silikons und des freien Silikons chemisch miteinander verbinden, um die Haftung des freien Silikons an dem dünnen aufpolymerisierten Silokonfilm zu verstärken, wobei der dünne aufpolymerisierte Silikonfilm eine Dicke von weniger als 1 Mikrometer, vorzugsweise zwischen 10 und 2000 Angström, aufweist.

2. Verfahren nach Anspruch 1, wobei der Schritt des Aufpolymerisierens das in Kontakt Bringen der Oberfläche, die mit dem fluiden Produkt in Kontakt steht, mit einer Lösung umfasst, die mindestens eine Haftungsgrundierung aufweist, wobei die Haftungsgrundierung ein spaltbares Arylsalz und mindestens ein Monomer oder ein Polymer ist, ausgewählt aus der Gruppe, die aus Siloxanen mit endständigen Vinyl- oder Acrylgruppen besteht.

3. Verfahren nach Anspruch 2, wobei die Siloxane mit endständigen Vinyl- oder Acrylgruppen ausgewählt sind aus der Gruppe bestehend aus Polyalkylsiloxanen mit endständigen Acryl- oder Vinylgruppen, wie Polymethylsiloxan mit endständigen Vinyl- oder Acrylgruppen, Polydimethylsiloxan mit endständigen Vinyl- oder Acrylgruppen, wie Polydimethylsiloxan-Acrylat (PDMS-Acrylat), Polyarylsiloxanen mit endständigen Vinyl- oder Acrylgruppen, wie Polyphenylsiloxan mit endständigen Vinyl- oder Acrylgruppen, wie Polyvinylphenylsiloxan, Polyarylalkylsiloxanen mit endständigen Vinyl- oder Acrylgruppen, wie Polymethylphenylsiloxan mit endständigen Vinyl- oder Acrylgruppen.

4. Verfahren nach Anspruch 2 oder 3, wobei das spaltbare Arylsalz ausgewählt ist aus der Gruppe bestehend aus Aryldiazoniumsalzen, Arylammoniumsalzen, Arylphosphoniumsalzen, Arylsulfoniumsalzen und Aryliodoniumsalzen.

5. Verfahren nach einem der vorhergehenden Ansprüche, wobei der zweite Schritt des Aufbringens von freiem Silikon durch Pufferung erfolgt.

## Claims

1. A surface treatment process of a metering valve, said metering valve comprising a valve body and a valve stem sliding in said valve body, **characterized in that** said process comprises a first step of forming a thin film of silicone by chemical grafting on at least one support surface of said valve body and/or said valve stem, and a second step of applying free silicone on said thin film of grafted silicone, such that said treated support surface simultaneously comprises grafted silicone and free silicone, siloxane chains of the grafted silicone and free silicone associating chemically together to reinforce the holding of the free silicone on said thin film of grafted silicone, said thin film of grafted silicone having a thickness less than 1 micrometer, preferably between 10 and 2000 angstroms

2. The process according to claim 1, wherein said grafting step comprises the contacting said surface in contact with the fluid product with a solution comprising at least one adhesion primer, said adhesion primer being a cleavable aryl salt and at least one monomer or a polymer selected from the group consisting of vinyl- or acrylic-terminated siloxanes.

3. The process according to claim 2, wherein vinyl- or acrylic-terminated siloxanes are selected from the group consisting of vinyl- or acrylic-terminated polyalkylsiloxanes such as vinyl- or acrylic-terminated polymethylsiloxane, vinyl- or acrylic-terminated polydimethylsiloxane such as polydimethylsiloxane-acrylate (PDMS-acrylate), vinyl- or acrylic-terminated polyarylsiloxanes such as vinyl- or acrylic-terminated polyphenylsiloxane such as polyvinylphenylsiloxane, vinyl- or acrylic-terminated polyarylalkylsiloxanes such as vinyl- or acrylic-terminated polymethylphenylsiloxane.

4. The process according to claim 2 or 3, wherein the cleavable aryl salt is selected from the group consisting of diazonium aryl salts, ammonium aryl salts, phosphonium aryl salts, sulfonium aryl salts and iodinium aryl salts.

5. The process according to any one of the preceding claims, wherein said second step of applying free silicone is conducted by buffering.
